# EUROPEAN PATENT APPLICATION

(11) **EP 1 952 803 A1**
(43) Date of publication of application: **06.08.2008**
(21) Application number: 07101047.4
(22) Date of filing: 23.01.2007
(51) Int. Cl.: A61K 9/00, A61K 9/127, A61K 9/16, A61K 9/28, A61K 9/48, A61K 31/045, A61K 31/121, A61K 31/683

(54) **Solid pharmaceutical dosage form containing hydrogenated phospholipids**

(71) Applicant: KTB-Tumorforschungs GmbH, 79106 Freiburg (DE)
(72) Inventor: Massing, Ulrich, 79249, Merzhausen (DE); Bauer-Brandl, Annette, 9014, Tromsoe (NO)
(74) Representative: Helbing, Jörg

(57) **Abstract**

The present invention provides a process for preparing a solid pharmaceutical dosage form (such as tablets, capsules and sachets) containing hydrogenated phospholipids and the solid pharmaceutical dosage form obtainable by such process.

## Description

The present invention provides a process for preparing a solid pharmaceutical dosage form (such as tablets, capsules and sachets) containing hydrogenated phospholipids and the solid pharmaceutical dosage form obtainable by such process.

### Background of the Invention

Recently, it was found that hydrogenated phospholipids (hereinafter "HPL"), perorally administered, can be therapeutically effective with respect to cancer disease and its subsequent effects. The HPL are particularly useful for the treatment of cancer cachexia, metastases and pain, as well as in treatment of inflammatory disease such as rheumatic arthritis, Morbus Crohn, multiple sclerosis, etc. (EP 05106747.8, PCT/EP2006/007775). Aqueous dispersions of the HPL were perorally as well as intraveneously administered in the respective experiments.

For the planning of clinical trials in humans, and the subsequent broader use of HPL for therapeutic purposes, these formulations (aqueous dispersions) are less advantageous as the dispersions contain water which induces chemical instability (hydrolysis) of the HPL during storage; and as HPL may sediment, which would prevent accurate dosing. This problem is most evident during storage, since the phase transition temperature of HPL is significantly above room temperature (phase transition temperatures for hydrogenated soy and egg HPL are at approx. 50 °C). Therefore, the HPL are in the crystalline state at room temperature, and would sediment even faster.

Peroral application of aqueous liposomal dispersions in such doses which are expected to be necessary for the therapeutical effect, would result in considerable volumes of aqueous dispersions to be taken in (in mouse experiments, 50 mg /kg/ day were used). This fact is expected to cause some practical problems, particularly in patients of reduced general health and nutrition status, who in many cases suffer from lack of appetite anyway (cancer related anorexia).

The total actual volume of the medicine a patient is supposed to take in should preferably be small, although the therapeutically effective dose is relatively high (in the range of some grams per day, depending on body weight and other factors). A small tablet with a high fraction of HPL seems most suitable. Preferably a minimum of 50 % of the mass of the tablet should be HPL. The HPL should rapidly disperse in the gastro-intestine in order to make enzymatic cleavage by phospholipase A2 into the respective lyso-phospholipids and fatty acids and subsequent absorption possible.

It is known from the literature, that particularly fast dissolution of drugs from a matrix (tablet) made of HPL is not possible. In contrast, HPLs are even used as excipients to retard tablets, where relatively small amount of HPL sustain the release of drug substance from the tablets (Nishihata T et al, Chem Pharm Bull (1987) 35, 4271-6). Furthermore, it is known from the literature that phospholipids (hereinafter "PL") used in large quantities in formulations in general obstruct processing by being sticky, soft, and plastic, and that problems with PL containing bulk materials occur regarding their flowability. These problems do not only refer to the production of tablets, but also other single dose application forms like capsules and sachets (sticking, variability of dosing). WO 01/13891 discloses that some phospholipids allow fast drug release, not from tablets, but from very small particles (some µm in diameter) when administered pulmonarily. It is shown that the modulation of the release rate is connected to the phase transition temperature of the phospholipids used. The phospholipids that give fastest release in pulmonal applications are those with low phase transition temperatures. Here, HPL has considerably higher phase transition temperature than body temperature, and release shall take place in the stomach where there is a lot more water present than in the lungs.

EP 1 543 841 discloses the preparation of small particles in the nanometer scale by dissolving in organic solvents and subsequent concentration. The preparations also contain surfactants and cyclodextrin in order to increase solubility of drugs. The following patents and patent applications refer to problems encountered when tabletting non-hydrogenated phospholipids:
GB 2 305 604 discloses that soy lecithin (powder) in amounts of 5% reduces capping.
US 4,882,167 relates to controlled release (slow release) by direct compression using hyrophobic carbohyrates (e.g. ethylcellulose) as a matrix. Lecithin is included to prevent capping.
US 4,762,658 discloses a method of tabletting of de-oiled phosphatides (lecithin). The de-oiled phosphatides are present in amounts of at least 80%. The moisture content is approximately 1% (which is achieved by drying the granular phospholipid powder) and the compressibility is < 15%. The lecithin should contain max. 1 % moisture, otherwise it is sticking. Granular lecithin is hygroscopic. At 68% relative humidity within hours it would reach up to 2.2 % moisture content.
WO 02/096354 discloses direct compression using lecithin as a binder, and the necessity of a disintegrant. Lecithin fractions in the tablets are: 0.5 - 99 % for the commercial product Centrolex FP 40 (available form Central Soya Inc., Iowa). Sodium-lauryl sulphate as a surfactant further helps to enhance oral bioavailablility.
EP 0 072 469 discloses a method of making a pharmaceutical and/or nutritional dosage form containing lecithin. Plasticity and cohesiveness is characteristic for lecithin. Said method (not tabletting or encapsulation) comprises extrusion and cutting into accordingly shaped pieces. As a disintegrant Avicel is utilized. Oil (0.5 - 5% ) is added to increase the plasticity of the material.
WO 97/36577 discloses solid lipid compositions of lipophilic compounds for enhanced oral bioavailability. The PL content 40%, dry solid lipid composition, contains at least one solid fat, plus more excipients (cryoprotectant, antioxidant, free flowing imparting agent, solid carrier, or diluent).
WO2005/023011 discloses a formulation based on phospholipids as the active ingredient, namely a granule having a diameter of about 70-500 µm that is homogeneously dispersible in water. The phospholipid content of the formulation is 5-70%, the rest is a fructane. The granule is prepared by a wet granulation process, which brings in 8% of water. The water content of the filled granule is 0.01%.
US 2004/00911535 discloses coated tablets where a phospholipid is used as a stabilizing agent in a coating for particles. The phospholipid is used as surface active substance. HPL is named to be less amenable to absorption of moisture. WO 00/33817 relates to a phospholipid compositions containing lipid and polymer (e.g. methacrylate) possessing high loading capacity of lipophilic and hydrophilic compounds. Manufacturing includes the use of organic solvents and a subsequent drying step. The waxy lipid materials can be compressed into tablets. WO99/32092 discloses a process for manufacturing a fast dispersion tablet that has at least one active ingredient. The tablet is prepared by roller compaction, slugging, dry granulation, milling and sieving. Phospholipids may be used as taste-masking agent.
US2005/0147672 discloses quickly disintegrating tablets having a phospholipid content < 20%.
JP63048226 discloses a slowly releasing solid preparation for oral administration. A slow release granule is prepared by kneading phospholipids with solvent (water, ethanol etc.), sieving, and finally tabletting.

However, the above findings with regard to PL do not apply to HPL. Namely, dry HPL, in contrast to phospholipids of marine and other biological origin like egg or say, is hard and elastic. Furthermore, and not less surprisingly, it was possible to make powder bulk materials containing considerable fractions of HPL of satisfying flowability. If this powder blend was tableted, capping was persistently observed, properties which may be traced back to the elastic properties of HPL. This problem would persist, even if larger fractions of other excipients are used. However, this method is disadvantageous regarding the size of the tablets with respect to the high dose of HPL to be used.

In view of the above, there was an urgent need to present an application form for hydrogenated phospholipid (HPL) which is pleasant and easy to take perorally, is single dosed, contains suitable amounts of HPL, releases the HPL fast, and is simple and cost effective to produce.

### Summary of the Invention

It was now found that the addition of water or other liquids (e.g. ethanol, acetone) to HPL in a granulation process changes the deformation properties of HPL from elastic to plastic. Surprisingly, only small amounts of water (ethanol, acetone) are necessary. Larger amounts result in sticking of the substance. HPL loaded with a suitable amount of water (ethanol, acetone) surprisingly appear as a material, which can be processed to a powder mass of suitable flowability using only a limited amount of further excipients. Using these steps, capping was completely eliminated.

Moreover, it was found that direct addition of the total amount of water to the powdered HPL is difficult, because high local concentrations occur. At the spots of high water activity, the powder would stick together irreversibly, and further homogeneous processing is not possible.

Limitation of the total amount of water and its careful addition is important to avoid local over-wetting and to produce homogeneous bulk material. By these precautions, it is possible to yield a homogeneous tablettable mixture just by a blending procedure (direct compression).

The invention thus provides
(1) a process for preparing a solid pharmaceutical dosage form containing a hydrogenated phospholipid (HPL) component and pharmaceutically acceptable carriers/excipients, said method comprises
   (a) mixing an appropriate amount of a wetting agent with the pharmaceutically acceptable carriers/excipients to prepare a premixture, said carriers/excipients being stable upon contact with the wetting agent,
   (b) adding said HPL component to said premixture of (a) and
   (c) preparing the solid dosage form by compression;
(2) a solid pharmaceutical dosage form obtainable by the process (1).

### Detailed Description of the Invention

The term "solid dosage" form according to the invention includes powder, tablet, capsule, sachet and the like. A tablet which is flat faced or concave is, however, preferred. Such tablet may have a diameter of 6-8 mm.

The solid dosage form of the invention contains the HPL component, hence the HPL, as the main component. It preferably has a HPL content from 25 to 85 % more preferably higher than 50% by weight, relative to the total weight of the solid dosage form.

The HPL component according to the invention comprises at least one HPL, namely a 1,2-diacylphospholipids containing predominantly saturated acyl residues or a pharmaceutically acceptable salt thereof. Such HPL possess an iodine number < 50, preferably < 10, most preferably < 5 and/or has the structure of formula (I) 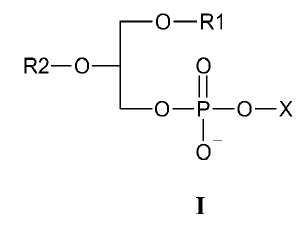 wherein
R¹ und R² are independently selected from H, alkylcarbonyl and arylalkylcarbonyl residues, wherein the alkyl residues are linear, branched or cyclic, saturated or unsaturated and may optionally be substituted with 1 to 3 residues R³ and one or more of the C-atoms in the alkyl residues may be substituted by O or NR⁴;
X is selected from H (the compound is then a phosphatid acid (PA)), -(CH₂)ₙ-N(R⁴)₃⁺ (this class of compounds comprises phosphatidylethanolamines (PE) and phosphatidylcholines (PC)), -(CH₂)ₙ-CH(N(R⁴)₃⁺)-COO⁻ (this class of compound comprises phosphatiodylserines (PS)) and -(CH₂)ₙ-CH(OH)-CH₂OH (this class of compounds comprises phosphatidylglycines (PG)), wherein n is an integer from 1 to 5;
R³, irrespective of the occurrence of further residues R³, is selected from H, lower alkyl (wherein the lower alkyl residues may be linear, branched or cyclic, saturated or unsaturated), F, Cl, CN and OH; and
R⁴, irrespective of the occurrence of further residues R⁴, is selected from H, CH₃ and CH₂CH₃,
or a pharmacologically acceptable salt thereof.

In particular, the residues R¹ and R² are alkylcarbonylresidues, preferably fatty acid residues, wherein linear (i.e. non-branched) fatty acid residues are particularly preferred. These fatty acid residues may be saturated or unsaturated and may have the same or a different length, particularly preferred is a chain length of C10 to C24, particularly preferred is a chain length of C14 to C24, most preferred of C16 to C24.

The HPL used in the process of the invention are those having a naturally occurring head in particular phosphatidylcholine, phosphatidylethanolamine, phosphatidylglycerine, phosphatidylserine and phosphatidacids, particularly preferred are HPLs belonging to the class of phosphatidylcholine. Phosphatidylcholine exclusively having saturated acyl residues are particularly preferred.

Particularly preferred are compounds of the formula (I), wherein R¹ und R² are independently H or linear and unsubstituted acyl residues, namely are saturated (alkylcarbonyl residues) and are preferably selected from lauroyl (n-dodecanoyl), myristoyl (n-tetradecanoyl), palmitoyl (n-hexadecanoyl), stearoyl (n-octadecanoyl), arachinoyl (n-eicosanoyl), behenoyl (n-docosanoyl) and lignoceroyl (n-tetracosanoyl) residues and are most preferably selected from myristoyl, palmitoyl, stearoyl and arachinoyl residues.

Particularly preferred are HPL containing predominantly saturated acyl residues, wherein greater than 50 %, preferably greater than 90 %, most preferably greater than 95% of the acl residues are saturated. The HPL can be produced synthetically or by hydrogenation of naturally occurring phospholipids, e.g. phospholipids previously isolated from eggs or soy. The HPL contain at least 20% phospatidylcholine, while a HPL content of more than 50 % or more than 70 % is particularly preferred (the remainder comprising among others phosphatidylethanolamines, other polar phospholipids, unsaturated phospholipids, lyso-phospholipids (such as lysoPC and lysoPE), free fatty acids, mono-, di- and triglycerides which might be saturated and unsaturated fatty acids or sterols (such as cholesterol, phytosterols like stigmasterin) or ester derivatives of the sterols).

Particular examples of the HPLs which are commercially available are the following products of the Fa. Lipoid, Ludwigshafen, Germany that are isolated from soy and egg:
Lipoid S75-3-N having a phosphatidylcholine and lyso-phosphatidylcholin content of at least 70 % and an iodine number < 3;
Lipoid S75-3 having a phosphatidylcholine and lyso-phosphatidylcholin content of 67-71 % and an iodine number < 3
Lipoid E PC-3 having a phosphatidylcholine content of at least 98% and an iodine number < 3.

The wetting agent used in the method of the invention is a polar solvent including water, ethanol, acetone, preferably is water. Such wetting agent is present in an amount of 0.1 to 10 % preferably 1 to 6 % by weight, relative to the total weight of the solid dosage form.

Carriers/excipients according to the invention include fillers, binders, glidants, disintegrants and lubricants.

Dry HPL is elastic, the tablets laminate and tend to cap. HPL takes up water and becomes sticky, tacky and ductile.

Disintegrants have to be added in order to prevent that the tablets become tacky upon contact with water but disintegrate so that the reactive agent (here predominantly the HPL) can be taken up. The disintegrant swells upon contact with water (more or less irreversibly) and the tablet disintegrates into small pieces. Tablets without disintegrant (e.g. being composed of HPL, Avicel and

Aerosil) become tacky upon contact with water and dissolve very slowly.

Further disintegrants include crosslinked polyvinylpyrrolidon, starchglycolat-Na, starches, sodium alginate, sodium carboxy methyl, celluloses, bentonite and others.

Microcrystalline cellulose is a filler/binder, some commercial qualities thereof can directly be tabletted. It adsorbs and desorbs water comparably easily. The intention for the preparation of a premixture is the homogenous distribution of the water in the premixture which provides controlled transition of water to the HPL (to therewith prevent tackyness).

Properties of suitable carriers/excipients for the premixture: Water can be distributed homogeneously without clotting and can desorb easily to transfer to HPL (the same applies mutatis mutandis for premixtures with sodium carbonate and citric acid, which react with each other and produce water)

Collodal SiO₂ is utilized for hydrophilisation of the mixture and for increasing the flowability of the mixture, which is important for automatic tabletting.

Glidants are unnecessary in most cases of tabletting HPL, but may be used. Glidants include aluminiumstearat, starch, polyethylene glycol and others.

Lubricants may optionally be added. Examples: Magnesium stearate, talcum, hydrogenated ricinus oil and the like.

The tabletting mass may include other common excipients used in tabletting (fillers, binders, disintegrants, glidants, lubricants, adsorption agents, wetting agents, disintegrants, hydrophilisisers, and antistatics). The tablets can also be flavoured and/or coloured if this should be suitable.

In a preferred embodiment of the process of the invention further carriers/excipients, preferably such carriers/excipients which are unstable upon contact with the wetting agent, as well as pharmaceutically active compounds including unsaturated phospholipids, flavors and/or colors, may be added
(i) either to the premixture in step (b) together with the HPL, or
(ii) in a separate step (b').

The carriers/excipients unstable upon contact with the wetting agent include disintegrants, preferably the disintegrants include croscarmellose-Na, crosslinked PVP and sodiumstarch glycolate. The addition of such disintegrants is particularly relevant for preparing disintegrating tablets with high content of HPL, preferably for preparing tablets with a HPL content of greater than 50% by weight.

Pharmaceutically active compounds include all kinds of pharmaceutically active substances, which are suitable to be tabletted. These substances include, but are not limited to agents active on the phospholipid metabolism (such as PLA2-inhibitors, COX-1/2/3-inhibitors, CYP2J2-Inhibitors (including imidazolyldodecanol) etc.), orally applicable cytostatic agents (such as alkyl phospholipids (APCs; including hexadecylphosphocholine (miltefonsin) and perifosin), alkylating agents/nitrosoureas (including Cyclophosphamid, Melphalan, Chlorambucil, Trofosfamid, Busulfan, Treosulfan), Procarbazin, etc.), antimetabolic agents (such as Methotrexat, Mercaptopurin, etc.), hormones (such as Medroxyprogesteronacetat (MPA), Megestrolacetat, Testolacton, Tamoxifen, Flutamid, Bicalutamid, Anastrozol, Letrozol, etc), and the like.

The unsaturated phospholipids are those included by the definition of formula I above, in particular compounds where R¹ and R² are unsaturated alkenyl- or alkinylcarbonyl residues. Particularly preferred compounds are those where R¹ and R² are selected from ω-3 und ω-9 fatty acid residues, in particular fatty acid residues derived from oleic acid (18:1), α-linolenic acid (18:3), eicosapentaenic acid (20:5; EPA) and docosahexaenic acid (22:6; DHA).

The invention will further be explained in connection with the following nonlimiting examples.

### Examples

### Materials and Methods (muss noch ergänzt werden)

HPL in the following examples is Lipoid S75-3N (Lipoid, D-Ludwigshafen).
As a blender the Turbula^{®} lab-scale power blender of Bachofen AG, Switzerland was utilized.
Tablets were compressed on a Diaf single-punch tablet presss (Diaf, Copenhagen, Denmark).
Crushing strength of the tablets was measured according to monograph 2.9.8.
Resistance to crushing of tablets, European Pharmacopoeia 5.06, 2007, on a ERWEKA TBH20 device (ERWEKA, Heusenstamm, Germany).

### Example 1 (Comparative Example):

HPL (5.0 g), Avicel PH101 (2.5 g), and Aerosil (0.37 g) were homogeneously blended in a lab-scale powder blender (Turbula^{®}). Tablets were compressed from the blend by hand, said tablets were smooth and suitably resistant to crushing. However, they did not disintegrate in water, but became tacky.

### Example 2 (Comparative Example):

HPL (25.0 g), Avicel PH101 (10.0 g), AcDiSol (2.5 g) and Aerosil (2.5 g) were homogeneously blended in a lab-scale powder blender (Turbula^{™}). Avicel PH102 (10.0 g) was added to yield the final blend. It was impossible to compress tablets from said blend due to capping problems.

### Example 3:

Procedure: Preparation of "Blend A": Avicel PH102 (150.0 g) and Water (18.3 g) were mixed until a homogeneous "Blend A" was obtained, which was stored in a tightly closed container prior to further use. HPL (200.0 g) and Blend A (126 g) was blended in a lab-scale powder blender for 5 min and subsequently sieved through 1000 µm sieve.

To the sieved material Croscarmellose-Na (AcDiSol ^{™}) (20.0 g) and Colloidal silicium dioxide (Aerosil^{™} A 200) (3.0 g), and optional 5 drops of lemon oil for better taste if suitable, was added and blended in lab scale powder blender for another 5 min to yield the final blend.

Tablets of 230± 5 mg, flat faced with break line diameter 8 mm were made.

The obtained tablets had a water content of 4.7 %, a phospholipid content of 57% (i.e. 130 mg HPL per tablet) and disintegrated in water within 15 min.

Crushing strengths of the tablets were in the range of 60-90N.

### Example 4:

"Blend B" was prepared by blending 30 g of sodium carbonate decahydrate and 200 g of HPL, blending 20 g of citric acid monohydrate and 50 g of HPL, and blending these together to yield 300 g of "Blend B".

Blend B (75.0 g), HPL (125.0 g) and Blend A (see example 3) (110.0 g) were blended in a lab-scale powder blender for 5 min.

Croscarmellose -Na (20.0 g) and Aerosil (2.5 g), and optional 5 drops of lemon oil for better taste if suitable, were added and blended in lab-scale powder blender for another 5 min to yield final blend.

Tablets of diameter 8 mm, 230 mg total mass were made which had a water content of 3.6 %, a lipid content of 56.4 % (i.e. 130 mg HPL per tablet) and disintegrated within 15 min. Crushing strengths of the tablets were in the range of 70-100N.

## Claims

1. A process for preparing a solid pharmaceutical dosage form containing a hydrogenated phospholipid (HPL) component and pharmaceutically acceptable carriers/excipients, said method comprises
(a)mixing an appropriate amount of a wetting agent with the pharmaceutically acceptable carriers/excipients to prepare a premixture, said carriers/excipients being stable upon contact with the wetting agent,
(b)adding said HPL component to said premixture of (a) and
(c) preparing the solid dosage form by compression.

2. The process of claim 1, wherein the solid dosage form is a powder, tablet, capsule or sachet, preferable is a small tablet being flat faced or concave, most preferably is a tablet having 6-8 mm diameter.

3. The process of claim 1 or 2, wherein the solid dosage form contains HPL as the active ingredient and as the main component, preferably has a HPL content from 25 to 85 % more preferably higher than 50% by weight, relative to the total weight of the solid dosage form.

4. The process of any one of claims 1 to 3, wherein said HPL component comprises at least one 1,2-diacylphospholipid containing predominantly saturated acyl residues or a pharmaceutically acceptable salt thereof, preferably said at least HPL having the structure of formula (I) 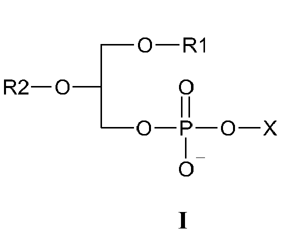 wherein
R¹ und R² are independently selected from H, alkylcarbonyl and arylalkylcarbonyl residues, wherein the alkyl residues are linear, branched or cyclic, saturated or unsaturated and may optionally be substituted with 1 to 3 residues R³ and one or more of the C-atoms in the alkyl residues may be substituted by O or NR⁴;
X is selected from H, -(CH₂)ₙ-N(R⁴)₃⁺, -(CH₂)ₙ-CH(N(R⁴)₃⁺)-COO⁻ and -(CH₂)ₙ-CH(OH)-CH₂OH (wherein n is an integer from 1 to 5);
R³, irrespective of the occurrence of further residues R³, is selected from H, lower alkyl (wherein the lower alkyl residues may be linear, branched or cyclic, saturated or unsaturated), F, Cl, CN and OH; and
R⁴, irrespective of the occurrence of further residues R⁴, is selected from H, CH₃ and CH₂CH₃,
or a pharmacologically acceptable salt thereof.

5. The process of any one of claims 1 to 4, wherein the wetting agent
(i) is a polar solvent including water, ethanol, acetone, preferably is water; and/or
(ii) is present in an amount of 0.1 to 10 % preferably 1 to 6 % by weight, relative to the total weight of the solid dosage form.

6. The process of any one of claims 1 to 5, wherein the carrier/excipient are selected from fillers, binders, glidants, disintegrants, lubricants.

7. The process of any one of claims 1 to 6, which comprises adding
(i) either to the premixture in step (b) together with the HPL, or
(ii) in a separate step (b')
further carriers/excipients, preferably such carriers/excipients which are unstable upon contact with the wetting agent.

8. The process of claim 7, wherein said carriers/excipients unstable upon contact with the wetting agent include disintegrants, preferably the disintegrants include croscarmellose-Na, crosslinked PVP or sodiumstarch glycolate.

9. The process of any one of claims 1 to 8, which further comprises adding further pharmaceutically active compounds in steps (a), (b), and/or (b'); flavors, colors, unsaturated phospholipids.

10. Solid pharmaceutical dosage form obtainable by the process of any one of claims 1 to 8.
